# EUROPEAN PATENT APPLICATION

(11) **EP 4 549 587 A1**
(43) Date of publication of application: **07.05.2025**
(21) Application number: 23306903.8
(22) Date of filing: 02.11.2023
(51) Int. Cl.: C12Q 1/6886, G01N 33/574

(54) **METHOD FOR THE DIAGNOSIS OF A CANCER WITH CHROMATIN-BOUND MDM2**

(71) Applicant: Institut Regional du Cancer de Montpellier, 34090 Montpellier (FR); Institut National de la Santé et de la Recherche Médicale, 75013 Paris (FR); UNIVERSITE DE MONTPELLIER, 34090 Montpellier (FR); Regen Lab France, 91940 Les Ulis (FR)
(72) Inventor: Linares, Laetitia, 34160 Saint Jean de Cornies (FR); Turzi, Antonino, 3782 Lauenen b. Gstaad (CH); Gadea, Gilles, 34730 Prades-le-Lez (FR); Riquier-Morcant, Blanche, 34080 Montpellier (FR); Firmin, Nelly, 34160 Saint Génies des Mourgues (FR)
(74) Representative: Valet Patent Services Limited

(57) **Abstract**

The disclosure relates to the *in vitro* use of the combined (i) measure of MDM2-encoding DNA and (ii) measure of interleukin 6 protein, in platelets, or in platelet-derived extracellular vesicles, of a subject, as a biomarker indicative of the occurrence of a cancer having chromatin-bound MDM2 in the said subject.

## Description

### FIELD OF THE DISCLOSURE

The present disclosure relates to the field of cancer diagnosis, especially of the diagnosis of cancers having chromatin-bound MDM2.

### BACKGROUND OF THE DISCLOSURE

Several cancers are caused by a disruption of the mouse double minute 2 (MDM2) oncoprotein. MDM2 is recognized as an essential component of the tumor suppressor p53 pathway that is frequently overexpressed in several types of human cancers (Biderman *et al*., 2012, Wade *et al*., 2013).

Liposarcoma is the second most common malignancy of soft tissues and a significant portion thereof is a liposarcoma having MDM2 bound to chromatin.

Recently, it has been demonstrated that MDM2 may be recruited to chromatin, independently of p53, to regulate a transcriptional program implicated in amino acid metabolism and more particularly in that of serine and glycine (Riscal et al. Mol Cell. 2016 Jun 16;62(6):890-902). MDM2, when bound to chromatin, operates independently of p53 to control serine/glycine metabolism and sustains cancer growth. Serine/glycine metabolism supports the growth of cancer cells by contributing to their anabolic demands as well as by regulating their redox state (Locasale JW. Nat Rev Cancer. 2013 Aug;13(8):572-83) and nucleotide synthesis (Cissé et al., Sci Transl Med. 2020 Jun 10;12(547).

MDM2 overexpression is a common occurrence in many types of cancer. By overexpressing MDM2, cancer cells have means to block p53. The sarcomas in which MDM2 amplification is a hallmark are well-differentiated liposarcomalatypical lipomatous tumor, dedifferentiated liposarcoma, intimal sarcoma and low-grade osteosarcoma.

The relatively low incidence and often atypical clinical presentation of soft tissue sarcomas impedes early and adequate diagnosis. Patients may report on recently enlarged soft-tissue swellings, infrequently complain of painful lesions, or even have no symptoms at all. A thorough diagnostic work-up is essential in order to distinguish between benign soft-tissue tumors, such as lipomas, and soft-tissue sarcomas, such as liposarcomas. Patient history, clinical features and radiological findings all help in assessing the underlying pathology.

Diagnosis can encompass imaging which helps confirming clinical findings by detecting soft-tissue mass, to estimate its size, tissue quality and relation to adjacent structures in detail, and to aid planning of the further course of action.

Collecting and analyzing a biopsy sample is an essential part of the diagnostic pathway, but invasive, which is generally practiced only after performing an imaging analysis, so as to avoid contaminating neurovascular structures or joints. Providing a biopsy sample allows performing histology, grading and staging analyses, with the view of determining which therapeutic intervention would likely be the most appropriate.

According to the applicant's knowledge, there are presently very few biomarkers indicative of the occurrence of a soft-tissue sarcoma in subjects.

Regarding more precisely liposarcoma, its diagnosis is of a further complexity because of the great clinical similarities with the benign affection of lipomas or atypical lipomatous tumors, for which complete resection is generally curative.

An illustration of the diagnosis methods that are presently contemplated is disclosed in the PCT application published under n° WO2019/106126, which relates to methods for the diagnosis of subjects suffering from liposarcoma exhibiting recruitment of MDM2 to chromatin or resistant to inhibitors of p53 and MDM2 interaction and discloses the diagnostic use of determining the level of serine and/or glycine in a subject's biological sample as well as the diagnostic use of determining the level of chromatin-bound MDM2 in a subject's biological sample. Thus, this document discloses recruitment of MDM2 to chromatin as an eligible biomarker for diagnosing liposarcomas.

Another illustration is disclosed in Casadei et al. (2019 Cancer Res., Vol. 79 (9) : 4911 - 4922), who disclosed that extracellular vesicles derived from patients affected with dedifferentiated liposarcoma and those derived from liposarcoma cell lines are carriers of MDM2 DNA that can be transferred to preadipocytes leading to impaired p53 activity in preadipocytes and increased proliferation, migration, and production of matrix metalloproteinase 2. These authors concluded that their results suggested that circulating extracellular vesicles containing MDM2-encoding DNA may serve as a biomarker, perhaps informing prognosis and facilitating early detection of dedifferentiated liposarcoma progression or recurrence, or possibly even predicting therapeutic resistance.

The results of Casadei et al. (2019, Cancer Res., Vol. 79 (9) : 4911 - 4922) thus showed that blood-derived extracellular vesicles from liposarcoma patients contained MDM2-encoding DNA, but with no information regarding the precise origin of such extracellular vesicles. Thus, these results cannot direct the medical practitioners to an appropriate medical anti-cancer treatment.

There is still a need in the art for method allowing diagnosis, especially early diagnosis, of the occurrence of a cancer having chromatin-bound MDM2.

### SUMMARY OF THE DISCLOSURE

The present disclosure relies on the transport by platelets, of a subject suspected to have a cancer cMDM2, of extracellular vesicles which have been loaded in the tumor microenvironment and which content includes, among others, IL6 protein and MDM2 DNA.

Otherwise said, the experimental results disclosed herein show that, in subjects affected with a cancer having MDM2 bound to the chromatin, platelets located at proximity of cancer cells, *e.g.* in the tumor microenvironment, are loaded with extracellular vesicles containing MDM2-encoding DNA as well as interleukin 6 protein originating from the tumor cells. This explains why these extracellular vesicles are also termed "platelet-derived" extracellular vesicles herein.

The present disclosure relates to an *in vitro* use of the combined (i) measure of MDM2-encoding DNA and (ii) measure of interleukin 6 protein in platelets, or platelet-derived extracellular vesicles, of a subject, as a biomarker indicative of the occurrence of a cancer having chromatin-bound MDM2 in the said subject.

It also pertains to an *in vitro* diagnostic method for determining the occurrence of a cancer having chromatin-bound MDM2 in a subject, comprising the steps of:
a) measuring (i) MDM2-encoding DNA and (ii) interleukin 6 protein in platelets, or platelet-derived extracellular vesicles, from a blood sample from the said subject, wherein the combined measures of MDM2-encoding DNA and of the interleukin 6 protein consist of a biomarker indicative of the occurrence of a cancer having chromatin-bound MDM2 in the said subject, and
c) concluding on the occurrence of a cancer having chromatin-bound MDM2 in the said subject, based on the values measured at step a)..

In some embodiments, the platelet-derived extracellular vesicles are obtained from a platelet-rich plasma sample or from a platelet-rich plasma sample containing activated platelets.

In some embodiments, the platelet-derived extracellular vesicles are obtained according to a method comprising the steps of :
a) providing platelet-rich blood plasma from a subject's whole blood sample,
b) performing a first ultracentrifugation step, so as to provide a sample depleted in macrovesicles,
c) performing, on the sample obtained at step b), a second ultracentrifugation step so as to provide a sample where extracellular vesicles are present in the pellet thereof, and
d) collecting the extracellular vesicles in the pellet obtained at step c).

In some embodiments, the platelet-derived extracellular vesicles are obtained from a sample of platelet-rich plasma, where the said extracellular vesicles are freely in suspension.

In some embodiments, determining (i) measuring MDM2-encoding DNA and (ii) measuring interleukin 6 protein, at step a) of the diagnostic method, is performed by using a platelet-rich plasma sample from the tested subject, which contains extracellular vesicles that have been released by the platelets contained therein.

In some embodiments, the platelet-derived extracellular vesicles are obtained from a platelet-rich plasma sample or from a platelet-rich plasma sample containing activated platelets.

In some embodiments of the method, the platelet-derived extracellular vesicles can be obtained from activated platelets.

In some embodiments of the *in vitro* diagnostic method, the tumor-derived extracellular vesicles are obtained by a method comprising the steps of:
i) obtaining platelet-rich plasma sample from a subject's whole blood sample, and
ii) activating the platelets contained in the blood plasma sample obtained at step i), so as to cause the release of the extracellular vesicles contained in the activated platelets in the platelet environment medium.

In some embodiments of the *in vitro* diagnostic method, the platelet-derived extracellular vesicles are obtained by a method comprising the steps of:
i) obtaining platelet-rich blood plasma sample from a subject's whole blood sample,
ii) activating the platelets contained in the blood plasma sample obtained at step i), so as to cause the release of the extracellular vesicles contained in the activated platelets in the platelet environment medium, and
iii) purifying the extracellular vesicles contained in the platelet environment medium obtained at step ii).

In some embodiments of the *in vitro* diagnostic method, MDM2-encoding DNA is measured by performing a DNA amplification reaction, such as a PCR reaction.

In some embodiments of the *in vitro* diagnostic method, interleukin 6 protein is measured by performing a method selected from an immunological method, a nuclear magnetic resonance (NMR) method or a method using mass spectrometry.

In some embodiments of the *in vitro* diagnostic method, interleukin 6 protein is measured by performing an immunological method, such as an ELISA method.

In some embodiments of the *in vitro* diagnostic method, the cancer having chromatin-bound MDM2 is selected among bone cancer, brain cancer, ovary cancer, breast cancer, lung cancer, colorectal cancer, osteosarcoma, skin cancer, malignant hemopathies, pancreatic cancer, prostate cancer and liposarcoma.

The present disclosure also concerns a therapeutic agent aimed at treating a cancer having chromatin-bound MDM2 for use in the treatment of a subject that has been determined as being affected with cancer having chromatin-bound MDM2 by performing the *in vitro* diagnostic method described herein.

In some embodiments of the said use, the said therapeutic agent is selected among an inhibitor of the MDM2-p53 interaction and an interleukin 6 signaling inhibitor.

In some embodiments of the said use, the said therapeutic agent is an interleukin 6 signaling inhibitor. The said interleukin 6 inhibitor can be selected among an anti-IL-6 antibody, an anti-IL-6 receptor antibody, an anti-IL6/IL-6 receptor complex and a gp130 inhibitor. The said gp130 inhibitor can be selected among an anti-gp130 antibody, bazedoxifene and an anti-sense oligonucleotide directed to gp130 mRNA.

In some embodiments, the said therapeutic agent is a MDM2 inhibitor.

The present disclosure further pertains to a kit for determining the occurrence of a cancer having chromatin-bound MDM2 in a subject comprising (i) means for measuring MDM2-encoding DNA and (ii) means for measuring interleukin 6 protein.

### DESCRIPTION OF THE FIGURES

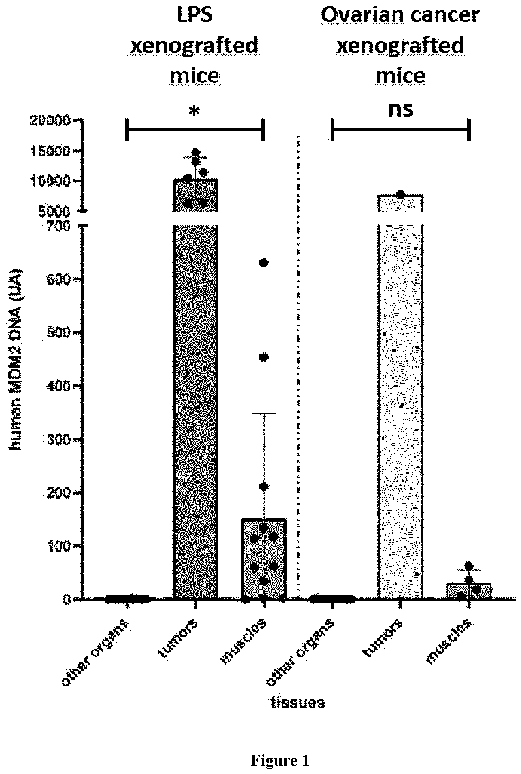

**Figure 1** illustrates the respective levels of MDM2-encoding DNA found in cancer cells derived from a human chromatin-bound MDM2 grafted tumor ("LPS" for liposarcoma) and in cancer cells derived from another human grafted tumor (human ovarian cancer).

Abscissa : left part of the figure : results from the human, patient-derived, liposarcoma-grafted mice. right part of the figure : results from the human, patient-derived, ovarian cancer. In each left or right part of the figure, from left to right: (i) other organs, (ii) tumor and (iii) muscles.

Ordinate : level of human MDM2 DNA, as expressed in arbitrary unites (UA).

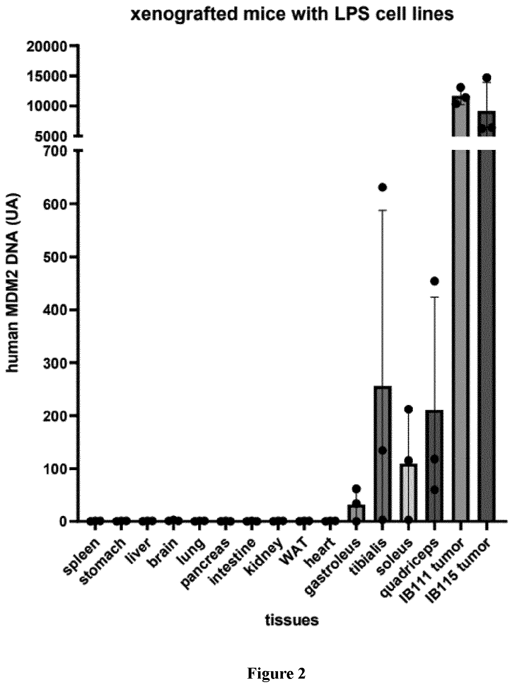

**Figure 2** illustrates the distribution of MDM2-encoding DNA in various tissues of mice xenografted with the human liposarcoma cell lines IB111 and IB115 (mean results obtained from xenografted mice with IB111 or IB115, respectively).

Abscissa : tissues, from left to right (i) spleen, (ii) stomach, (iii) liver, (iv) brain, (v) lung, (vi) pancreas, (vii) intestine, (viii) kindney, (ix) WAT [White Adipose Tissue, (x) heart, (xi) gastroleus, (xii) tibiais, (xiii) soleus, (xiv) quadriceps, (xv) cell line IB111 xenograft and (xvi) cell line IB115 xenograft.

Ordinate ; content in human MDM2-DNA, as expressed in arbitrary units (AU).

**Figure 3** illustrates the MDM2 DNA content found in extracellular vesicles found respectively in extracellular vesicles derived from a human chromatin-bound MDM2 grafted tumor (tumor cell line IB115 a human liposarcoma) and in cancer cells derived from a human non chromatin-bound MDM2 grafted tumor (tumor cell line ZR75.1, a human ductal carcinoma cell line).

Figure 3A : results from the liposarcoma grafted tumor - IB115. Figure 3B : results from the human ductal carcinoma tumor - ZR75.1.

Bars in abscissa, from left to right : (i) no treatment ("unstressed"), (ii) induction of the localization of MDM2 to chromatin ("stressed").

Ordinate ; content in human MDM2-DNA, as expressed in arbitrary units (AU).

**Figure 4** illustrates the diameter size profile of the extracellular vesicles.

Abscissa ; extracellular vesicle diameter. Ordinate : number of extracellular particles

**Figure 5** illustrates the content in human MDM2-encoding DNA in extracellular vesicles contained in the tumor interstitial fluids (TIF) from cancerous subjects.

Abscissa, from left to right : (i) Control subjects affected from sarcomas without chromatin-bound MDM2, (ii) subjects affected with a liposarcomas having chromatin-bound MDM2.

Ordinate ; content in human MDM2-encoding DNA, as expressed in arbitrary units (AU).

**Figure 6** illustrates the content in human MDM2-encoding DNA in serum-derived extracellular vesicles from cancerous subjects.

Abscissa, from left to right : (i) Control subjects affected from sarcomas without chromatin-bound MDM2, (ii) subjects affected with a liposarcomas having chromatin-bound MDM2.

Ordinate ; content in human MDM2-encoding DNA, as expressed in arbitrary units (AU).

**Figure 7** illustrates the content in IL-6 protein in platelets from mice that have been xenografted with human liposarcoma cells, abscissa : within each group of bars, from left to right : (i) platelets-containing plasma, (ii) activated platelets-containing plasma, (iii) activated platelets-containing serum. Each group of bars disclose a single xenografted mouse.

**Figure 8** illustrates the content in interleukin 6 (IL-6) from various human cancer cell lines.

Figure 8A : content in interleukin 6-encoding mRNA (IL-6 mRNA) from various human cancer cell lines. Ordinate : relative content in human IL-6-encoding mRNA, as expressed in arbitrary units (a.u.).

Figure 8B : content in interleukin 6-encoding protein (IL-6) of supernatants from various human cancer cell lines. Ordinate : Concentration in IL-6 protein, as expressed in ng/mL).

Abscissa, from left to right : (i) IB115 liposarcoma cell line, (ii) IB111 liposarcoma cell line, (iii) SKOV3 ovarian adenocarcinoma cell line, (iv) HPAC pancreatic ductal adenocarcinoma cell line, (v) ZR-75-1 ductal breast cell line and (vi) CFPAC-1 ductal pancreatic adenocarcinoma cell line.

### DETAILED DESCRIPTION

### Definitions

The terms used in the present specification generally have their ordinary meaning in the art. Certain terms are discussed below, or elsewhere in the present disclosure, to provide additional guidance in describing the products and methods of the presently disclosed subject matter.

The following definitions apply in the context of the present disclosure.

As used in this specification and the appended claims, the singular forms "a", "an" and **"the"** include plural referents unless the content clearly dictates otherwise.

The term **"about"** or **"approximately"** means within an acceptable error range for the particular value as determined by one of ordinary skill in the art, which will depend in part on how the value is measured or determined, e.g., the limitations of the measurement system. For example, "about" can mean within 1 or more than 1 standard deviation, per the practice in the art. Alternatively, "about" can mean a range of up to 10% of a given value. Alternatively, particularly with respect to biological systems or processes, the term can mean within an order of magnitude, preferably within 5-fold, and more preferably within 2-fold, of a value. Where particular values are described in the application and claims, unless otherwise stated the term "about" meaning within an acceptable error range for the particular value should be assumed.

It is understood that aspects and embodiments of the present disclosure described herein include **"comprising", "having",** and **"consisting of,"** aspects and embodiments. The words "have" and "comprise," or variations such as "has," "having," "comprises," or "comprising," will be understood to imply the inclusion of the stated element(s) (such as a composition of matter or a method step) but not the exclusion of any other elements. The term "consisting of" implies the inclusion of the stated element(s), to the exclusion of any additional elements. The term "consisting essentially of" implies the inclusion of the stated elements, and possibly other element(s) where the other element(s) do not materially affect the characteristic(s) of the stated elements. It is understood that the different embodiments of the disclosure using the term "comprising" or equivalent cover the embodiments where this term is replaced with "consisting of" or "consisting essentially of'.

The term **"MDM2"** has its general meaning in the art and refers to mouse double minute 2 oncoprotein. The term "MDM2" also refers to E3 ubiquitin-protein ligase and human MDM2 has the UniProtKB accession number Q00987.

As used herein, the term **"Interleukin-6"** (IL-6) refers to a protein involved in the regulation of immune responses such as lymphocyte differentiation. IL-6 is well known in the art and is also known as B cell-stimulating factor 2 (BSF-2) or interferon beta-2 (IFNB2). The IL-6 gene encodes a 212 amino acid polypeptide and the naturally occurring human IL-6 protein has an aminoacid sequence as shown in Uniprot Accession number P05231. Naturally occurring human IL-6 variants are known and included in this definition. For human interlukin-6 gene, it may be referred to the NIH Accession number Gene ID : 3569.

As used herein, **"extracellular vesicles"** encompass membranous vesicles derived from cells that appear in tissue fluids of different sources, including blood, urine, cerebrospinal fluid, pleural fluid, malignant ascites, breast milk, amniotic fluid, birth canal fluid, gastrointestinal fluid, bronchoalveolar Lavage fluid and saliva. Extracellular vesicles can be formed by a donor cell (*e.g*., a cancer cell). Afterwards, a receptor cell (*e.g.* a megakaryocyte) or a non-nucleated body components (*e.g.* blood platelets) can absorb the released extracellular vesicles through endocytosis, membrane fusion, or specific ligand-receptor internalization The vesicles are used to deliver the contents of extracellular vesicles (for example, nucleic acids, lipopolysaccharides, proteins and/or lipids) from the donor cell to the recipient cell. It is known that extracellular vesicles mediate different physiological and pathological responses, including organ development, cell-to-cell communication, tumor metastasis, and immune-related diseases (for example, autoimmune diseases, degenerative diseases, or inflammatory diseases). Thus, the term "extracellular vesicle" refers to a cell-derived vesicle comprising a membrane that encloses an internal space. Extracellular vesicles comprise all membrane-bound vesicles that have a smaller diameter than the cell from which they are derived. Generally extracellular vesicles range in diameter from 20 nm to 1000 nm, and can comprise various macromolecular cargo either within the internal space, displayed on the external surface of the extracellular vesicle, and/or spanning the membrane. The cargo can comprise nucleic acids, proteins, carbohydrates, lipids, small molecules, and/or combinations thereof. By way of example and without limitation, extracellular vesicles include vesicles derived from cells, such as cancer cells, or cell-derived membrane-containing components, such as blood platelets, by direct or indirect manipulation. Extracellular vesicles can be derived from a living or dead organism, explanted tissues or organs, and/or cultured cells. In the particular context of the present disclosure, "extracellular vesicle" mainly means vesicles that are derived from platelets. These platelet-derived extracellular vesicles, originating from subject's cancer cells, were absorbed by blood platelets and then transported by these blood platelets, and are possibly at least partly released by platelets in the blood fluid.

As used herein, the term **"subject"** refers to a mammal. Mammals include, but are not limited to, domesticated animals (e.g., cows, sheep, cats, dogs, and horses), primates (e.g., humans and non-human primates such as monkeys), rabbits, and rodents (e.g., mice and rats). Preferably, the subject refers to a human. As used herein, **"subject in need thereof"** refers to a living organism suffering from or prone to a cancer disease or condition that can be treated by a method according to the present disclosure. Typically, a subject in need thereof according to the disclosure refers to any subject, preferably a human, afflicted with or susceptible to be afflicted with a cancer exhibiting recruitment of MDM2 to chromatin, in particular liposarcoma. Typically, a subject according to the present disclosure refers to any subject, preferably a human, afflicted with or susceptible to be afflicted with a liposarcoma. In some embodiments, the term "subject" refers to any subject afflicted with or susceptible to be afflicted with cancer types exhibiting recruitment of MDM2 to chromatin, including liposarcoma, ovarian cancers, glioblastoma, breast cancers, melanoma, colorectal cancers, kidney cancers, bone cancer, brain cancer, skin cancer, malignant hemopathies, AML (Acute myeloid leukemia), pancreatic cancer, prostate cancer, and lung cancer or cancer exhibiting exacerbated serine and glycine metabolism, including advanced melanoma and lung cancer.

The terms **"cancer having chromatin-bound MDM2", "cancer expressing chromatin-bound MDM2", "cancer exhibiting recruitment to chromatin", "C-MDM2"** and **"cancer having MDM2 localized at the chromatin"** can be used interchangeably herein and means a cancer wherein MDM2 is localized in the cell nucleus. Without wishing to be bound by any particular theory, it is specified that a cancer exhibiting recruitment of MDM2 to chromatin does not equate to cancer exhibiting a MDM2 overexpression in the cell. A cancer exhibiting recruitment of MDM2 to chromatin encompass both (i) cancers exhibiting overexpression of MDM2 and (ii) cancers that do not exhibit MDM2 overexpression. A cancer exhibiting recruitment of MDM2 to chromatin may be a cancer wherein the cancer cells exhibit a MDM2 overexpression in the cytoplasm and wherein at least a part of the cellular MDM2 is localized in the cell nucleus. A cancer exhibiting recruitment of MDM2 to chromatin may be a cancer wherein MDM2 is not overexpressed in the cancer cells and wherein at least part of the cellular MDM2 is localized in the cell nucleus. Accordingly, in some embodiments, a cancer exhibiting recruitment of MDM2 to chromatin may not be a cancer exhibiting a MDM2 overexpression in the cytoplasm.

The term **"liposarcoma"** or **"LPS"** has its general meaning in the art and refers to soft tissue sarcomas of mesenchymal origin such as revised in the World Health Organisation Classification ICD10 C49.9. The term "liposarcoma" also refers to well-differentiated and dedifferentiated liposarcoma (WD- and DD-LPS). The term "liposarcoma" also relates to Malignant mesenchymal neoplasms, a type of soft tissue sarcoma, a group of lipomatous tumors of varying severity ranging from slow-growing to aggressive and metastatic. Liposarcomas are most often located in the lower extremities or retroperitoneum, but they can also occur in the upper extremities, neck, peritoneal cavity, spermatic cord, breast, vulva and axilla. The term "liposarcoma" also relates to Dedifferentiated liposarcoma and Well-differentiated liposarcoma. In some embodiments, the term "liposarcoma" refers to liposarcoma exhibiting recruitment of MDM2 to chromatin and MDM2-mediated control of serine metabolism independently of p53. In some embodiments, the term "liposarcoma" refers to liposarcoma resistant to inhibitors of p53 and MDM2 protein interaction.

The term **"expression"** when used in the context of expression of a gene or nucleic acid refers to the conversion of the information, contained in a gene, into a gene product. A gene product can be the direct transcriptional product of a gene (e.g., mRNA, tRNA, rRNA, antisense RNA, ribozyme, structural RNA or any other type of RNA) or a protein produced by translation of a mRNA. Gene products also include messenger RNAs, which are modified, by processes such as capping, polyadenylation, methylation, and editing, and proteins (e.g., IL-6, IL-6 receptor, STAT3 or gp130) modified by, for example, methylation, acetylation, phosphorylation, ubiquitination, SUMOylation, ADP-ribosylation, and glycosylation.

An **"expression inhibitor"** refers to a natural or synthetic compound that has a biological effect to inhibit the expression of a gene, such as to also inhibit the production of a protein when the said gene consists of a protein-encoding gene.

The term **"MDM2 inhibitor"** refers to compounds that selectively block or inactivate the chromatin function of MDM2. As used herein, the term "selectively block or inactivate the chromatin function of MDM2" refers to a compound that preferentially binds to and block or inactivate the effect or function of MDM2 on chromatin with a greater affinity and potency, respectively, than other ubiquitin-protein ligases, or related enzymes or related transporters. Compounds that block or inactivate the chromatin function of MDM2 may also block or inactivate, enzymes related to PHGDH, PSAT, PSPH, or other members of the SLC1 family of proteins, as partial or full inhibitors, are contemplated. Typically, a MDM2 inhibitor is a small organic molecule, a polypeptide, an aptamer, an antibody, an intra-antibody, an oligonucleotide or a ribozyme. The MDM2 inhibitors are well-known in the art as illustrated by (Qin et ah, 2016; US8329723). MDM2 inhibitor may be selected from the group consisting of SP141, 6-Methoxy-l-naphthalen-2-yl-9H-P-carboline; SP141 nanoparticles (SP141NP); SPl4l-loaded IgG Fc-conjugated maleimidyl-poly(ethylene glycol)-co-poly(e- caprolactone) (Mal-PEG-PCL) nanoparticles (SPl4lFcNP); 6-Mcthoxy- 1 -quinolin-4-yl-9H-P- carboline; 6-Methoxy- 1 -naphthalen- 1 -yl-9H-P-carboline; 6-Methoxy- 1 -phenanthren-9-yl-9H- b-carboline; 7-Mcthoxy- 1 -phcnanthrcn-9-yl-9H-P-carbolinc; miR-509-5p; shRNA and compounds described in Qin et ah, 2016 ; US8329723.

The term **"PHGDH"** refers to phosphoglycerate dehydrogenase having the UniProtKB accession number 043175, Catalyzes the oxidation of 3 -phosphoglycerate (3PG) to 3-phosphohydroxypyruvate (3P-Pyr), the first step of the serine biosynthesis pathway.

The term **"Interleukin-6 (IL-6) signaling inhibitor"** refers to compounds or agents that selectively block or inactivate the IL-6 signaling pathway. As used herein, the term "selectively blocks or inactivates" refers to a compound that preferentially bind to and block or inactivate IL-6, IL-6 receptor, IL-6/IL-6R complex, STAT3 and/or gp130. Especially compounds that block the interaction between IL-6 with its IL-6 receptor, IL-6 with gp130, IL-6 receptor with gp130, IL-6/IL-6 Receptor complex with gp130, or compounds that block the phosphorylation of STAT3 Typically, an IL-6 signaling inhibitor may encompass without limitation a polypeptide, an aptamer, an antibody or a portion thereof, an antisense oligonucleotide, *i.e*., a siRNA or a shRNA, or a ribozyme. Such types of inhibitors are further described in the present description. As used herein, the term "IL-6 signaling inhibitors" of the present disclosure encompass compounds selected in the group comprising (i) compounds blocking the interaction between IL-6 and IL-6 receptor, such as compounds that selectively block IL-6 recruitment to its IL-6 receptor with the consequence to inhibit the expression of genes involved in serine metabolism including phosphoglycerate dehydrogenase (PHGDH), phosphohydroxythreonine aminotransferase (PSAT) and phosphoserine phosphatase (PSPH), (ii) compounds blocking the interaction between IL-6 and gp130, (iii) compounds blocking the interaction between IL-6 receptor and gp130, (iv) compounds blocking the interaction between IL-6/IL-6R complex with gp130, and (v) compounds blocking the phosphorylation of STAT3 after the activation of the receptor of IL-6.

The terms **"IL-6/IL-6R complex"** or **"IL-6/IL-6 receptor complex"** collectively refer to a complex formed by a soluble form of IL-6 and membrane-bound form IL-6 receptor alpha. In some embodiments of the present disclosure, **"IL-6/IL-6R complex"** refers to a complex formed by (i) a soluble form of IL-6 secreted by a cancer cell of a subject, and (ii) a membrane-bound form IL-6 receptor alpha of a myoblast cell of the same subject.

As used herein, **"administering"** or **"administered"** means administration by any route, such as oral administration, administration as a suppository, topical contact, parenteral, intraperitoneal, intramuscular, intralesional, intrathecal, intranasal, subcutaneous or transmucosal (*e.g*., buccal, sublingual, palatal, gingival, nasal, vaginal, rectal, or transdermal) administration, or the implantation of a slow-release device, *e.g*., a mini-osmotic pump, to a subject, including parenteral. Parenteral administration includes intravenous, intramuscular, intra-arteriole, intradermal, subcutaneous, intraperitoneal, intraventricular, and intracranial.

As used herein, **"pharmaceutically"** or **"pharmaceutically acceptable"** refers to molecular entities and compositions that do not produce an adverse, allergic or other untoward reaction when administered to a mammal, especially a human, as appropriate. A pharmaceutically acceptable carrier or excipient refers to a non-toxic solid, semi-solid or liquid filler, diluent, encapsulating material or formulation auxiliary of any type. By way of example of pharmaceutically acceptable carrier, sterile water, saccharides such as sucrose or saccharose, starches, sugar alcohols such as sorbitol, polymers such as PVP or PEG, lubricating agents, such as magnesium stearate, preservatives, dyeing agents or flavors can be mentioned.

As used herein, the terms **"prevent"** or **"preventing"** with respect to a disease or disorder relate to prophylactic treatment of a cancer disease, e.g., in an individual suspected to have the cancer disease, or at risk for developing the cancer disease. Prevention may include, but is not limited to, preventing or delaying onset or progression of the cancer disease and/or maintaining one or more symptoms of the cancer disease at a desired or sub-pathological level. The term "prevent" does not require the 100% elimination of the possibility or likelihood of occurrence of the event. Rather, it denotes that the likelihood of the occurrence of the event has been reduced in the presence of a composition or method as described herein. More particular, "prevent" or "preventing" refers to a decrease in the risk of occurrence of a cancer disease or symptom in a patient. As indicated above, the prevention may be complete, i.e., no detectable symptoms or disease, or partial, such that fewer symptoms or less severity of the disease are observed than would likely occur absent treatment.

The terms **"treat"** or **"treatment"** or **"therapy"** in the present disclosure refer to the administration or consumption in a subject in need thereof of an IL-6 signaling inhibitor or a pharmaceutical composition comprising an IL-6 signaling inhibitor of the present disclosure, optionally in combination with a serine/glycine deprivation of cancer cells according to the present disclosure, with the purpose to cure, heal, alleviate, relieve, alter, remedy, ameliorate, improve, or affect a cancer disorder as described herein, the symptoms of the condition, or to prevent or delay the onset of the symptoms, complications, or otherwise arrest or inhibit further development of the cancer disorder. More particularly, "treating" or "treatment" includes any approach for obtaining beneficial or desired results in a subject's cancer condition. The treatment may be administered to a subject having a cancer exhibiting recruitment of MDM2 to chromatin or who ultimately may acquire the cancer exhibiting recruitment of MDM2 to chromatin. Beneficial or desired clinical results can include, but are not limited to alleviation or amelioration of one or more cancer symptoms or conditions, diminishment or reduction of the extent of a cancer disease or of a cancer symptom, stabilizing, *i.e*., not worsening, the state of a cancer disease or of a cancer symptom, prevention of a cancer disease or of a cancer symptom's spread, delay or slowing of cancer disease or cancer symptom progression, amelioration or palliation of the cancer disease state, diminishment of the reoccurrence of cancer disease, and remission, whether partial or total and whether detectable or undetectable. In other words, "treatment" as used herein includes any cure, amelioration, or reduction of a cancer disease or symptom. A "reduction" of a symptom or a disease means decreasing of the severity or frequency of the disease or symptom, or elimination of the disease or symptom.

By a **"therapeutically effective amount"** of the MDM2 inhibitor or of a IL-6 signaling inhibitor of the present disclosure is meant a sufficient amount of the IL-6 signaling inhibitor for treating cancer at a reasonable benefit/risk ratio applicable to any medical treatment. The specific amount that is therapeutically effective can be readily determined by an ordinary medical practitioner and may vary depending on factors such as the type and stage of pathological processes considered, the subject's medical history and age, and the administration of other therapeutic agents. The specific therapeutically effective dose level for any particular subject will depend upon a variety of factors including the disorder being treated and the severity of the disorder; activity of the specific inhibitor employed; the specific composition employed, the age, body weight, general health, sex and diet of the subject; the time of administration, route of administration, and rate of excretion of the specific inhibitor employed; the duration of the treatment; drugs used in combination or coincidential with the specific inhibitor employed; and like factors well known in the medical arts. For example, it is well within the skill of the art to start doses of the MDM2 inhibitor or the IL-6 inhibitor at levels lower than those required to achieve the desired therapeutic effect and to gradually increase the dosage until the desired effect is achieved. However, the daily dosage of the products may be varied over a wide range from 0.01 to 1,000 mg per adult per day. Typically, the compositions may contain 0.01, 0.05, 0.1, 0.5, 1.0, 2.5, 5.0, 10.0, 15.0, 25.0, 50.0, 100, 250 or 500 mg of MDM2 inhibitor or of IL-6 signaling inhibitor of the present disclosure for the symptomatic adjustment of the dosage to the subject to be treated. A medicament typically contains from about 0.0002 mg to about 500 mg of the selected MDM2 inhibitor or of the IL-6 signaling inhibitor of the present disclosure, preferably from 0.01 mg to about 100 mg of the MDM2 inhibitor or of the IL-6 signaling inhibitor of the present disclosure. An effective amount of the IL-6 signaling inhibitor is ordinarily supplied at a dosage level from about 0.0002 mg/kg to about 500 mg/kg of body weight per day, especially from about 0.01 mg/kg to about 100 mg/kg, and in particular from about 0.1 mg/kg to 50 mg/kg of body weight per day.

### Detailed description

The inventors have unexpectedly found that the presence of both MDM2-encoding DNA and interleukin 6 protein (also termed "IL-6" herein) in a platelet sample, especially in a platelet-rich sample from a blood sample previously obtained from a subject, especially of a subject affected with a cancer, is indicative of a cancer having MDM2 bound at the chromatin, such as a liposarcoma having chromatin-bound MDM2.

As it is shown in the examples herein, MDM2-encoding DNA is found in various cancer cells, *i.e.* in cancer cells wherein MDM2 DNA is amplified and wherein MDM2 is overexpressed, which confirm the available data in the prior art.

However, MDM2-encoding DNA is found in a significant amount only, in platelets, and especially in extracellular vesicles originating from cancer cells and absorbed by platelets of subjects affected with a cancer constitutively having chromatin-bound MDM2.

Further, when a tumor that is constitutively devoid of chromatin-bound MDM2 is induced to localize MDM2 at the chromatin, then MDM2-encoding DNA can be found in the platelets thereof, including in the extracellular vesicles transported by the said platelets. These results show that the presence of MDM2-encoding DNA in platelets of a subject, including in platelet-derived extracellular vesicles transported by platelets of a subject, contributes discriminating between (i) a cancer having MDM2 bound chromatin and (ii) a cancer devoid of chromatin-bound MDM2.

Further, the inventors have found that, specifically in subjects affected with a cancer having MDM2 bound to the chromatin, such as subjects affected with a liposarcoma having chromatin-bound MDM2, platelets, and the platelets-derived extracellular vesicles that are found in the subjects' platelets, also contain interleukin 6 (IL-6) protein. Conversely, in subjects affected with a cancer devoid of chromatin-bound MDM2, no IL-6 protein is found in platelets nor in these platelet-derived extracellular vesicles.

The `platelet-derived" extracellular vesicles found in the platelets of cancer subjects were identified as being derived from the cancer cells of the said cancer subjects since, especially in mice xenografted with human cancer cells, only human MDM2-encoding DNA and human IL-6 protein are found therein, as it shown in the examples.

Altogether, the inventors' findings show that the presence of both MDM2-encoding DNA and IL-6 protein in platelets, including in tumor-derived extracellular vesicles originating from platelets of a cancer subject, is indicative that the said cancer subject is affected by a cancer having chromatin-bound MDM2, such as a liposarcoma having chromatin-bound MDM2.

Importantly, these inventors' findings enable a very early detection of the occurrence of a cancer having MDM2 bound to the chromatin in subjects, the said early detection allowing increased chances of success for a therapeutic treatment which can be provided to the said subject at a time period wherein the tumor is of a small size and has not yet generated metastasis.

Further, these inventors' findings are particularly useful since they allow more easily determining an appropriate therapeutic treatment, such as a therapeutic treatment more specifically adapted against cancers having MDM2 bound to chromatin, which treatments are illustrated elsewhere in the present specification.

The present disclosure relates to the *in vitro* use of the combined (i) measure of MDM2-encoding DNA and (ii) measure of interleukin 6 protein in platelets, or in platelet-derived extracellular vesicles of a subject as abiomarker indicative of the occurrence of a cancer having chromatin-bound MDM2 in the said subject.

This disclosure relates to an *in vitro* diagnostic method for determining the occurrence of a cancer having chromatin-bound MDM2 in a subject, comprising the steps of:
a) measuring (i) MDM2-encoding DNA and (ii) interleukin 6 protein, in platelets, or in platelet-derived extracellular vesicles from a blood sample from the said subject, wherein the combined measures of MDM2-encoding DNA and interleukin 6 protein consist of a biomarker indicative of the occurrence of a cancer having chromatin-bound MDM2 in the said subject, and
b) concluding on the occurrence of a cancer having chromatin-bound MDM2 in the said subject, based on the values measured at step a)..

Taken together, the MDM2-encoding DNA value and the interleukin 6 protein value that are measured at step a) are used as a biomarker indicative of the occurrence of a cancer having chromatin-bound MDM2 in the said subject.

In preferred embodiments of step a), measuring MDM2-encoding DNA in a sample means measuring the amount of MDM2-encoding DNA in the said sample, irrespective of the measuring amount units that are used.

In preferred embodiments of step a), measuring interleukin 6 protein in a sample means measuring the amount of interleukin 6 protein in the said sample, irrespective of the measuring amount units that are used.

In preferred embodiments of step b), the measuring values of MDM2-encoding DNA and of interleukin 6 protein that are obtained at step a) are compared to reference values. Most preferably, at step b), (i) the measuring value of MDM2-encoding DNA is compared with a reference value of MDM2-encoding DNA and (ii) the measuring value of interleukin 6 protein is compared with a reference value of interleukin 6 protein.

Various reference values can be used at step b) of the *in vitro* diagnostic method. Reference values are most preferably already previously available when performing the *in vitro* diagnostic method according to the present disclosure. For example, a reference value which is used according to the *in vitro* diagnostic method disclosed herein can be the mean value of a collection of values, such as a collection of MDM2-encoding DNA values or a collection of interleukin 6 protein values, previously obtained in either (i) subjects who are not affected with a cancer and subjects who are affected with a cancer which is devoid of chromatin-bound MDM2 or (ii) in subjects who are affected with a cancer having chromatin-bound MDM2, such as subjects who are affected with a liposarcoma having chromatin-bound MDM2.

In some embodiments, the reference values are those derived from the measure of MDM2-encoding DNA and interleukin 6 protein in subjects who are not affected with a cancer having chromatin-bound MDM2, which encompasses subjects who are not affected with a cancer and subjects who are affected with a cancer which is devoid of chromatin-bound MDM2. In these embodiments, step b) comprises concluding on the occurrence of a cancer having chromatin-bound MDM2 in the said subject when the value of MDM2-encoding DNA and the value of interleukin 6 protein measured at step a) are both higher than the respective reference values.

It is in the background knowledge of the skilled artisan to determine when a measured value is higher than a reference value, in the context of the parameter which is measured and according to the measuring method which is used.

According to preferred embodiments of the present disclosure, a measured amount value is considered as being higher than a reference amount value when the measured amount value is of about twice or more higher than the reference amount value, irrespective of whether MDM2-encoding DNA or interleukin 6 protein is considered.

In most preferred embodiments of the *in vitro* diagnostic method, a measured value of MDM2-encoding DNA, such as a measured amount of MDM2-encoding DNA, is higher than the corresponding reference value when the said measured value is about **five times** or more higher than the reference value of MDM2-encoding DNA, such as the reference amount value of MDM-2-encoding DNA that can be found in the same type of sample obtained from subjects affected with a cancer which has not MDM2 bound to the chromatin or subjects who are not affected with a cancer. The measured value of MDM2-encoding DNA can be expressed in any kind of Units, such as in Arbitrary Units (UA), as disclosed in the examples herein. Illustratively, when a reference value of MDM2-encoding DNA is of about 100 arbitrary units (*e.g*. UA) in a control sample, then a higher value of MDM2-encoding DNA in a test sample is concluded when the amount of MDM2-encoding DNA measured in the said test sample is about 500 arbitrary units or more.

In most preferred embodiments of the *in vitro* diagnostic method, a measured value of interleukin 6 protein, such as a measured amount of interleukin 6 protein, is higher than the corresponding reference value of interleukin 6 protein when the said measured value that is about **twice** or more higher than the reference value of interleukin 6 protein, such as the reference amount value of interleukin 6 protein that is found in the same type of sample obtained from control subjects, such as control subjects affected with a cancer which has not MDM2 bound to the chromatin or subjects who are not affected with a cancer. The amount of interleukin 6 protein can be expressed in any kind of units, such as in weight by volume (w/v), *e.g*. in ng/ml, as disclosed in the examples herein. Illustratively, when a background amount of interleukin 6 protein is of about 10 pg/ml in a control sample, then a higher value of interleukin 6 protein in a test sample is concluded when the amount of interleukin 6 protein measured in the said test sample is about 20 pg/ml or more.

In some embodiments, the reference values are those derived from the measure of MDM2-encoding DNA and interleukin 6 protein in subjects who are affected with a cancer having chromatin-bound MDM2, such as subjects who are affected with a liposarcoma having chromatin-bound MDM2. In these embodiments, step b) comprises concluding on the occurrence of a cancer having chromatin-bound MDM2 in the said subject when the value of MDM2-encoding DNA and the value of interleukin 6 protein measured at step a) are about the same as the respective reference values. According to the present disclosure, a measured value of MDM2-encoding DNA or interleukin 6 protein is "about the same" as the corresponding reference value when the measured value is at most 50% higher or at most 50% lower than the said reference value, irrespective of whether MDM2-encoding DNA or interleukin 6 protein is considered.

At step a) of the *in vitro* method above, the platelet-derived extracellular vesicles encompass those which are spontaneously released by platelets as well as those which are released after platelet activation.

Generally, the said subject is a mammal subject, including a canine such as a dog, and is most preferably a human subject.

Thus, the MDM2-encoding DNA is a mammal MDM2-encoding DNA, including a canine MDM2-encoding DNA such as a dog MDM2-encoding DNA, and is most preferably a human MDM2-encoding DNA. It is reminded that human MDM2 protein and encoding DNA can be found in the UniProt database under the UniProtKB accession number Q00987.

Also, interleukin 6 protein is a mammal interleukin 6, including a canine interleukin 6 such as a dog interleukin 6, and is most preferably a human interleukin 6 protein. It is specified that human Interleukin 6 protein and encoding DNA can be found in the UniProt database under the UniProtKB accession number P05231.

In some embodiments, when the presence of MDM2-encoding DNA and/or IL-6 is measured in platelets, the sample is a platelet-containing plasma sample.

In some preferred embodiments, when the presence of MDM2-encoding DNA and/or IL-6 is measured in platelets, the sample is the platelet-rich fraction of a plasma sample, *i.e.* is a platelet-rich plasma sample (also termed "PRP" in the art).

In some embodiments, the presence of MDM2-encoding DNA and/or IL-6 is measured in platelets, PRP, Bone Marrow Concentrate (BMC), platelet-derived extracellular vesicles, PRP-derived extracellular vesicles, BMC-derived extracellular vesicles, or any combination thereof.

Platelet sample, under the form of a platelet-rich fraction of a plasma sample can be obtained by any known method, well known from the skilled in the art. An illustrative embodiment is disclosed in the examples, wherein a whole blood sample is collected in a glass container containing an anticoagulant substance, such as sodium citrate, and a thixotropic gel that allows separating the platelet-containing plasma fraction from the blood cells, including the red blood cells by centrifugation and collecting the platelet-rich plasma fraction, *e.g.* with a syringe.

### Platelet-derived, extracellular vesicles

The platelet-derived extracellular vesicles originating from the subject's platelets can be obtained according to any method well known by the skilled artisan.

In preferred embodiments, the platelet-derived extracellular vesicles are obtained according to a method comprising the step of providing subject's platelet-containing plasma sample from a previously collected subject's blood sample. Any known method can be used by the skilled artisan.

According to these preferred embodiments, platelet-derived extracellular vesicles can be separated from the rest of the platelet-containing sample by a two-step ultracentrifugation method, as it is disclosed in the examples herein.

In some of these preferred embodiments, the platelet-derived extracellular vesicles can be obtained by a method comprising the steps of :
a) providing platelet-rich blood plasma from a subject's whole blood sample,
b) performing a first ultracentrifugation step, so as to provide a sample depleted in macrovesicles,
c) performing, on the sample obtained at step b), a second ultracentrifugation step so as to provide a sample where extracellular vesicles are present in the pellet thereof, and
d) collecting the extracellular vesicles in the pellet obtained at step c).

In some embodiments of the method above the centrifugation at step b) is performed at about 10 000 g, such as for about 30 minutes, such as at about 4°C

In some of these preferred embodiments, the platelet-derived extracellular vesicles can be obtained by a method comprising the steps of :
a) providing platelet-rich blood plasma from a subject's whole blood sample, and
b) performing a first ultracentrifugation step at about 10 000 g, such as for about 30 minutes, such as at about 4°C, so as to provide a sample depleted in macrovesicles,
c) performing, on the sample obtained at step b), a second ultracentrifugation step at about 100 000 g, such as for about 180 minutes, such as at about 4°C, and
d) collecting the extracellular vesicles in the cell pellet obtained at step c).

An extracellular vesicle-containing sample is thus obtained at the end of step d), which can be used for measuring MDM2-encoding DNA and IL-6 protein therein.

In some other embodiments, the platelet-derived extracellular vesicles originating from platelets can be obtained from a platelet-containing sample wherein the platelets have been previously activated.

Thus, in some other embodiments, the platelet-derived extracellular vesicles can be obtained by a method comprising the steps of:
a) providing platelet-rich blood plasma from a subject's whole blood sample,
b) activating the platelets contained in the blood plasma obtained at step a), so as to cause the release of the extracellular vesicles contained in the activated platelets in the platelet environment medium,
c) performing a first ultracentrifugation step, so as to provide a sample depleted in macrovesicles. In some embodiments, the said first ultracentrifugation step can be performed at about 10 000 g, such as for about 30 minutes, such as at about 4°C, so as to provide a sample depleted in macrovesicles,
d) performing, on the sample obtained at step c), a second ultracentrifugation step so as to provide a sample where extracellular vesicles are present in the pellet thereof. In some embodiments, the said second ultracentrifugation step can be performed at about 100 000 g, such as for about 180 minutes, such as at about 4°C. , and
e) collecting the extracellular vesicles in the cell pellet obtained at step d)

An extracellular vesicle-containing sample is obtained at the end of step e), which can be used for measuring MDM2-encoding DNA and IL-6 protein therein.

In some embodiments of step a) of any of the methods above, the platelet-rich plasma sample has been previously added with prostaglandins (prostin) so as to block platelet aggregation.

In some embodiments of step a) of any of the methods above for obtaining platelet-derived extracellular vesicles, the platelet-rich plasma sample has been previously added with an Acid-Citrate-Dextrose buffer to block coagulation.

At step b) of the method above, platelet activation can be performed by using chemical (CaCl2, ADP ..) or physical methods (centrifugation, agitation, warming, etc.). Most preferably, platelets are activated by incubation in a liquid solution using 10% CaCl₂-H₂O ated at room-temperature for 20 min and then centrifuged at 2300 rpm for 10 min.

In still other embodiments, the platelet-derived extracellular vesicles can be obtained according to a method comprising the steps of:
a) providing a platelet-rich plasma sample from the subject to be tested,
b) centrifuging the said platelet-rich plasma sample so as providing a supernatant fraction containing the extracellular vesicles, and
c) collecting the extracellular vesicles contained in the supernatant faction obtained at step b).

In some embodiments of step a), the platelet-rich plasma sample has been previously added with prostaglandins (prostin) so as to block platelet aggregation.

In some embodiments of step a), the platelet-rich plasma sample has been previously added with an Acid-Citrate-Dextrose buffer to block coagulation.

At step b), plasma can be centrifuged by differential centrifugation at a centrifugal force ranging from 2000 g to 2100 g, which can correspond to a centrifugation at a rotational speed of 2200 rpm when using a rotor having 350 mm diameter. In some embodiments, this centrifugation step is of a duration of about 10 minutes. In some embodiments, at the end of the centrifugation step, the rotational speed is let decreasing without using a brake.

Thus, in some embodiments of the method, the tumor-derived extracellular vesicles originate from activated platelets.

In some other embodiments, the tumor-derived extracellular vesicles are obtained by a method comprising the steps of :
i) obtaining platelet-rich blood plasma from a subject's whole blood sample,
ii) activating the platelets contained in the blood plasma obtained at step i), so as to cause the release of the extracellular vesicles contained in the activated platelets in the platelet environment medium, and
iii) purifying the extracellular vesicles contained in the platelet environment medium obtained at step ii).

### Measuring MDM2-encoding DNA

In most preferred embodiments, MDM2-encoding DNA is measured by quantifying MDM2-encoding DNA in a sample.

Quantifying DNA from vesicles can be performed by any method which is known from the skilled artisan. Vesicle DNA can even be analyzed at the single-vesicle level by nano-flow cytometry as described by Haisheng et al. (2022, J Extracell Vesicles, Vol. 11 (4) : e 12206).

For quantifying MDM2-encoding DNA contained in the tumor-derived extracellular vesicles originating from the subject's platelets, whole DNA is first extracted from the extracellular vesicles before quantifying the MDM2-encoding DNA, preferably by using a known DNA amplification method.

Thus, in some embodiments, MDM2-encoding DNA is measured by performing a DNA amplification reaction, such as by a quantitative PCR method.

Extracellular vesicle lysis reagents and procedures are known in the art and can generally be performed by chemical (for example, detergent, hypotonic solutions, enzymatic procedures, and the like, or a combination thereof), physical (for example, press French, ultrasonic, and the like) or electrolytic. Any suitable lysis procedure can be used. For example, chemical methods generally employ lysing agents to disrupt cells and extract nucleic acids from cells, followed by treatment with chaotropic salts. High salt content lysis procedures are also commonly used. For example, an alkaline lysis procedure can be used. Traditionally, the latter procedure incorporates the use of phenol-chloroform solutions, and an alternative non-phenol-chloroform procedure involving three solutions can be used. In these latter procedures, a solution may contain 15 mM Tris, pH 8.0; 10 mM EDTA and 100 ug/ml Rnase A; a second solution may contain 0.2 N NaOH and 1% SDS; and a third solution may contain 3M KOAc, pH 5.5. These procedures can be found in Current Protocols in Molecular Biology, John Wiley & Sons, N.Y., 6.3.1-6.3.6 (1989).

Then, the MDM2-encoding DNA possibly present in the platelet-originating, tumor-derived, extracellular vesicles, which can contain other nucleic acids, is subjected to a procedure that linearly or exponentially generates amplicon nucleic acids having the same nucleotide sequence as the target MDM2-encoding DNA, or a segment thereof.

In some embodiments, the MDM2-encoding DNA is amplified by performing a method comprising a polymerase chain reaction (PCR) with an appropriate MDM2-specific pairs of nucleic acid primers and a MDM2 amplicon-specific probe for detection and quantification. In most preferred embodiments, MDM2-encoding DNA contained in the platelet-originating, tumor-derived, extracellular vesicles is quantified by performing a quantitative polymerase chain reaction (qPCR) with an appropriate MDM2-specific pair of primers and a MDM2 amplicon-specific probe for detection and quantification.

In some embodiments, quantifying MDM2-encoding DNA can be performed by amplification using a qPCR method with an appropriate pair of primers designed from the MDM2 DNA and then quantifying the MDM2-encoding DNA with an amplicon-specific probe.

In some embodiments, quantifying MDM2-encoding DNA can be performed by amplification using a qPCR method with the pair of primers of **SEQ ID NO. 1** and **SEQ ID NO.** 2 and then quantifying the MDM2-encoding DNA with the probe of **SEQ ID NO. 3.**

In some embodiments, measuring measuring MDM2-encoding DNA in a platelet sample, or in platelet-derived extracellular vesicles, can be performed by the method known as the droplet digital polymerase chain reaction (also termed "dd PCR"), such as disclosed for example in EP 3663411.

### Measuring IL-6 protein

In most preferred embodiments, interleukin 6 protein is measured by quantifying interleukin 6 protein in a sample.

As shown in the examples, it was found that tumor-derived vesicles originating from platelets obtained from a subject affected with a cancer having MDM2 bound to the chromatin contain IL-6 protein.

Quantifying IL-6 protein present in extracellular vesicles can be performed by any method well known from the skilled artisan.

Quantifying IL-6 protein can be performed by using an immunological method, by a nuclear magnetic resonance (NMR) method or a method using mass spectrometry/

In some embodiments, quantifying IL-6 protein is performed by an immunological method. A plurality of commercial kits is available for detecting and quantifying IL-6 protein, including human IL-6 protein, in a sample.

In some embodiments, quantifying IL-6 protein can be performed by the conventional method of immunoblotting, by using IL6-binding detectable compounds, typically enzymatically or fluorescently labeled anti-IL-6 antibodies. Such methods generally comprise the steps of (i) lysing the extracellular vesicles to release proteins contained therein, followed by (ii) either (1) direct spotting on a membrane (dot blot assay) and detection using labeled anti-IL-6 antibodies or (2) separation of the protein using SDS-PAGE and detection using labeled anti-IL-6 antibodies.

Illustratively, the skilled artisan can use the commercial kit available from Abcam company under the reference number "ab 178013".

### Cancers having chromatin-bound MDM2

Cancers having chromatin-bound MDM2, that can be diagnosed with the method according to the present disclosure, may be of a large variety of types and thus can encompass the following cancers, provided that these cancers have been determined to consist of SLC1A4-expressing cancers : Acute Lymphoblastic Leukemia (ALL), Acute Myeloid Leukemia (AML), Adrenocortical Carcinoms, Childhood cancers, AIDS-Related Cancers, Kaposi Sarcoma, AIDS-Related Lymphoma, Primary CNS Lymphoma, Anal Cancer, Astrocytomas, Atypical Teratoid/Rhabdoid Tumor, Basal Cell Carcinoma, Skin Cancer (Nonmelanoma), Bile Duct Cancer, Bladder Cancer, Bone Cancer, Ewing Sarcoma Family of Tumors, Osteosarcoma and Malignant Fibrous Histiocytoma, Brain Stem Glioma, Atypical Teratoid/Rhabdoid Tumor, Embryonal Tumors, Germ Cell Tumors, Craniopharyngioma, Ependymoma, Breast Cancer, Bronchial Tumors, Burkitt Lymphoma, Non-Hodgkin Lymphoma, Carcinoid Tumor, Gastrointestinal Carcinoma, Cardiac (Heart) Tumors, Primary Lymphoma, Cervical Cancer, Cholangiocarcinoma, Chordoma, Chronic Lymphocytic Leukemia (CLL), Chronic Myelogenous Leukemia (CML), Chronic Myeloproliferative Neoplasms, Colon Cancer, Colorectal Cancer, Craniopharyngioma, Cutaneous T-Cell Lymphoma, Mycosis Fungoides and Sezary Syndrome, Ductal Carcinoma In Situ (DCIS), Embryonal Tumors, Endometrial Cancer, Ependymoma, Esophageal Cancer, Esthesioneuroblastoma, Extracranial Germ Cell Tumor, Extragonadal Germ Cell Tumor, Eye Cancer, Intraocular Melanoma, Retinoblastoma, Fallopian Tube Cancer, Fibrous Histiocytoma of Bone, Malignant, and Osteosarcoma, Gallbladder Cancer, Gastric (Stomach) Cancer, Gastrointestinal Carcinoid Tumor, Gastrointestinal Stromal Tumors (GIST), Germ Cell Tumor, Ovarian, Testicular, Gestational Trophoblastic Disease, Glioma, Hairy Cell Leukemia, Head and Neck Cancer, Hepatocellular (Liver) Cancer, Histiocytosis, Langerhans Cell, Hodgkin Lymphoma, Hypopharyngeal Cancer, Islet Cell Tumors, Pancreatic Neuroendocrine Tumors, Kaposi Sarcoma, Kidney, Renal Cell, Langerhans Cell Histiocytosis, Laryngeal Cancer, Leukemia, Acute Lymphoblastic (ALL), Acute Myeloid (AML), Chronic Lymphocytic (CLL), Chronic Myelogenous (CML), Hairy Cell, Lip and Oral Cavity Cancer, Liver Cancer (Primary), Lung Cancer, Non-Small Cell, Small Cell, Lymphoma, Hodgkin, Non-Hodgkin, Macroglobulinemia, Waldenstrom, Male Breast Cancer, Melanoma, Merkel Cell Carcinoma, Mesothelioma,Metastatic Squamous Neck Cancer with Occult Primary, Midline Tract Carcinoma Involving NUT Gene, Mouth Cancer, Multiple Endocrine Neoplasia Syndromes, Multiple Myeloma/Plasma Cell Neoplasm, Mycosis Fungoides, Myelodysplasia Syndromes, Myelodysplastic/Myeloproliferative Neoplasms, Myelogenous Leukemia, Chronic (CML), Myeloid Leukemia, Acute (AML) Myeloma, Multiple, Myeloproliferative Neoplasms, Nasal Cavity and Paranasal Sinus Cancer, Nasopharyngeal Cancer, Neuroblastoma, Non-Hodgkin Lymphoma, Non-Small Cell Lung Cancer, Oral Cancer, Oral Cavity Cancer, Lip and Oropharyngeal Cancer, Osteosarcoma and Malignant Fibrous Histiocytoma of Bone, Ovarian Cancer, Low Malignant Potential Tumor, Pancreatic Cancer, Pancreatic Neuroendocrine Tumors (Islet Cell Tumors), Papillomatosis, Paraganglioma, Paranasal Sinus and Nasal Cavity Cancer, Parathyroid Cancer, Penile Cancer, Pharyngeal Cancer, Pheochromocytoma, Pituitary Tumor, Plasma Cell Neoplasm/Multiple Myeloma, Pleuropulmonary Blastoma, Pregnancy and Breast Cancer, Primary Central Nervous System (CNS) Lymphoma, Primary Peritoneal Cancer, Prostate Cancer, Rectal Cancer, Renal Cell (Kidney) Cancer, Renal Pelvis and Ureter, Transitional Cell Cancer, Retinoblastoma, Rhabdomyosarcoma, Salivary Gland Cancer, Rhabdomyosarcoma, Uterine, Small Intestine Cancer, Soft Tissue Sarcoma, Sqamous Cell Carcinoma, Squamous Neck Cancer with Occult Primary, Metastatic, Ttomach (Gastric) Cancer, T-Cell Lymphoma, Testicular Cancer, Throat Cancer, Thymoma and Thymic Carcinoma, Thyroid Cancer, Transitional Cell Cancer of the Renal Pelvis and Ureter, Unknown Primary, Ureter and Renal Pelvis, Transitional Cell Cancer, Urethral Cancer, Uterine Cancer, Endometrial, Uterine Sarcoma, Vaginal Cancer, Vulvar Cancer, Waldenstrom Macroglobulinemia, and Wilms Tumor.

Cancers expressing chromatin-bound MDM2 (C-MDM2), *i.e.* cancers exhibiting recruitment of MDM2 to chromatin, that can be diagnosed with the method according to the present disclosure, are more particularly selected from the group consisting of bone cancer, brain cancer, ovary cancer, breast cancer, lung cancer, colorectal cancer, osteosarcoma, skin cancer, malignant hemopathies, including acute myeloid leukemia, pancreatic cancer, prostate cancer and liposarcoma and even more particularly selected from the group consisting of skin cancer, liposarcoma and blood cancer, for example but not limited to liposarcoma, melanoma and acute myeloid leukemia.

### Therapeutic uses

In various aspects, in the course of the determination of the occurrence of a cancer having chromatin-bound MDM2 with an *in vitro* diagnostic method disclosed herein, analysis of a subject's cancer cell-derived exosomes can be made over a particular time course in various intervals to assess a subject's progression and pathology. For example, analysis may be performed at regular intervals such as one day, two days, three days, one week, two weeks, one month, two months, three months, six months, or one year, in order to track the occurrence or confirmation of cancer having chromatin-bound MDM2 in order to provide useful information to medical practitioners in making appropriate therapeutic choices. In all cases, performing the of both MDM2-encoding DNA and of IL-6 protein in the tumor-derived extracellular vesicles originating from platelets according to a method disclosed herein, can provide an early warning that the patient has developed a Chromatin-bound MDM2 tumor, thus providing an early diagnosis.

The present disclosure relates to a therapeutic agent aimed at treating a cancer having chromatin-bound MDM2 for use in the treatment of a subject that has been determined as being affected with cancer having chromatin-bound MDM2 by performing the *in vitro* diagnostic method for determining the occurrence of a cancer having chromatin-bound MDM2 in a subject, according to the present disclosure.

The present disclosure relates to a method for treating a cancer subject in need thereof comprising the steps of:
1) performing the *in vitro* diagnostic method for determining the occurrence of a cancer having chromatin-bound MDM2 in a subject, disclosed herein,
2) providing a therapeutic treatment against a cancer having a chromatin-bound MDM2 to the said subject when the occurrence of a cancer having a chromatin-bound MDM2 has been determined at step 1).

The present disclosure relates to a method for treating a cancer subject in need thereof comprising the steps of:
1) performing the method for determining the occurrence of a cancer having chromatin-bound MDM2 in a subject, comprising the steps of:
   a) determining (i) the presence of MDM2-encoding DNA and (ii) the presence of interleukin 6 protein, in platelet-derived extracellular vesicles transported by platelets from a blood sample previously obtained from the said subject, and
   b) concluding on the occurrence of a cancer having chromatin-bound MDM2 in the said subject when the said sample is positive for both MDM2 and IL6, and
2) providing a therapeutic treatment against a cancer having a chromatin-bound MDM2 to the said subject when the occurrence of a cancer having a chromatin-bound MDM2 has been determined at step 1).

At step 2) of the method above, any therapeutic treatment against a cancer having a chromatin-bound MDM2 can be used.

In some embodiments, the said therapeutic treatment can comprise an active agent consisting of an inhibitor of the MDM2-p53 interaction, such as disclosed for example in the PCT application published under n° WO2016/049453.

In some embodiments, the said therapeutic treatment can comprise an active agent consisting of an inhibitor of the IL-6 signaling pathway.

In some embodiments, the said therapeutic treatment comprises an active agent comprising i) an inhibitor of the MDM2-p53 interaction and/or ii) an inhibitor of the IL-6 signaling pathway.

Inhibitors of the Interleukin-6 (IL-6) signaling pathway can be selected from an IL-6 inhibitor, an IL-6 receptor inhibitor, an IL-6/IL-6 receptor complex inhibitor, a gp130 inhibitor and a STAT3 inhibitor.

The term "Interleukin-6 (IL-6) signaling inhibitor" refers to compounds or agents that selectively block or inactivate the IL-6 signaling pathway. As used herein, the term "selectively blocks or inactivates" refers to a compound that preferentially binds to and blocks or inactivate IL-6, IL-6 receptor, IL-6/IL-6R complex, STAT3 and/or gp130. Especially compounds that block the interaction between IL-6 with its IL-6 receptor, IL-6 with gp130, IL-6 receptor with gp130, IL-6/IL-6 Receptor complex with gp130, or compounds that blocks the phosphorylation of STAT3. Typically, an IL-6 signaling inhibitor may encompass without limitation a polypeptide, an aptamer, an antibody or a portion thereof, an antisense oligonucleotide, i.e., a siRNA or a shRNA, or a ribozyme.

The terms "IL-6/IL-6R complex" or "IL-6/IL-6 receptor complex" collectively refer to a complex formed by a soluble form of IL-6 and membrane-bound form IL-6 receptor alpha. In some embodiments, "IL-6/IL-6R complex" refers to a complex formed by (i) a soluble form of IL-6 secreted by a cancer cell of a subject, and (ii) a membrane-bound form IL-6 receptor alpha of a myoblast cell of the same subject.

In some embodiments, the IL-6 signaling inhibitor can be selected among an anti-IL-6 antibody, an anti-sense oligonucleotide directed to IL-6 mRNA, an anti-IL-6 receptor antibody, an anti-sense oligonucleotide directed to IL-6 receptor mRNA, an anti-IL6/IL-6 receptor complex and a gp130 inhibitor.

In some embodiments, the IL-6 signaling inhibitor can be an antibody, or an antigen-binding portion thereof, directed against one of IL-6, IL-6 receptor, STAT 3 and/or gp130.

In some other embodiments, the IL-6 signaling inhibitor may be an IL-6 expression inhibitor, an IL-6 receptor expression inhibitor, a STAT 3 expression inhibitor and/or a gp130 expression inhibitor.

In some embodiments, expression inhibitors for use in the method according to the present disclosure may be anti-sense oligonucleotides.

In some embodiments, the IL-6 signaling inhibitor of the present disclosure may be a chemical compound.

In some embodiments, a chemical compound of the present disclosure includes compounds inhibiting the IL-6 expression, IL-6R expression, STAT expression, the phosphorylation of STAT3 and/or gp 130 expression.

In some embodiments, the A therapeutic agent for use according to claim 10, wherein the gp130 inhibitor is selected among an anti-gp130 antibody, bazedoxifene and an anti-sense oligonucleotide directed to gp130 mRNA.

In some embodiments, chemical compounds that can be used in combination with an anti-xlSLC1A4 monoclonal antibody of the present disclosure may be blocking anti-gp130 compounds, compounds that inhibit the phosphorylation of STAT3 or compounds that inhibit the expression of STAT3.

Some of those blocking anti-gp130 compound are illustrated by, for instance, Wu et al., Mol Cancer Ther. 2016 Nov;15(11):2609-2619.

In some embodiments, a gp-130 inhibitor of the present disclosure may be bazedoxifene,

In some embodiments, a STAT3 inhibitor may be selected from SH5-07, APTSTAT3-9R, C188-9, BP-1-102, Niclosamide (BAY2353), STAT3-IN-1, WP1066, Cryptotanshinone, Stattic, Resveratrol (SRT501), S31-201, HO-3867, Napabucasin (BBI608), Brevilin A, Artesunate (WR-256283), Bosutinib (SKI-606), TPCA-1, SC-43, Ginkgolic acid C17:1, Ochromycinone (STA-21), Colivelin, Cucurbitacin Iib, GYY4137, Scutellarin, Kaempferol-3-O-rutinoside, Cucurbitacin I, SH-4-54, Nifuroxazide and Pimozide.

In some embodiments, a STAT3 inhibitor may be C188-9, Stattic or a combination thereof.

As used herein "bazedoxifene" has its general meaning in the art and consists of a gp130 inhibitor having the formula: 1-[[4-[2-(Hexahydro-1H-azepin-1-yl)ethoxy]phenyl]methyl]-2-(4-hydroxyphenyl)-3-methyl-1H-indol-5-ol monoacetate (salt) with the molecular formula C30H34N2O3 • C2H4O2 and the CAS Number 198481-33-3.

As used herein "C188-9" is a potent inhibitor of STAT3 that binds to STAT3 with high affinity. C188-9 may be defined by the reference: CAS No. 432001-19-9.

As used herein "Stattic" is a small molecule inhibiting STAT3 activation. Stattic may be defined by the reference: CAS No. 19983-44-9.

In some embodiments, the A therapeutic agent is a MDM2 inhibitor.

### Kits

The present disclosure also relates to a kit for determining the occurrence of a cancer having chromatin-bound MDM2 in a subject comprising (i) means for determining the presence of MDM2-encoding DNA and (ii) means for determining the presence of interleukin 6 protein.

Means for determining the presence of MDM2-encoding DNA are preferably selected among those already described elsewhere in the present specification.

Means for determining the presence of interleukin 6 protein are preferably selected among those already described elsewhere in the present specification.

The kit can thus contain oligonucleotides for use in amplifying a MDM2-encoding DNA and/or a target segment of a MDM2-encoding DNA contained in the tumor-derived extracellular vesicles.

Also, the kit can thus contain an IL-6 binding molecule, especially an IL-6 antibody for use for detecting and/or quantifying IL-6 contained in the tumor-derived extracellular vesicles.

In preferred embodiments of a kit is according to the present disclosure, the said kit can further contain one or more of the necessary components to prepare a platelet-rich plasma from a whole blood sample.

In some other embodiments of a kit is according to the present disclosure, the said kit can further contain one or more of the necessary components to collect extracellular vesicles from a body fluid, most preferably from a platelet-rich plasma.

The kit may comprise one or more sealed vials containing any of such components. In some embodiments, the kit may also comprise a suitable container means, which is a container that will not react with components of the kit, such as an Eppendorf tube, an assay plate, a syringe, a bottle, or a tube. The container may be made from sterilizable materials such as plastic or glass.

The kit may further include an instruction sheet that outlines the procedural steps of the methods set forth herein, and will follow substantially the same procedures as described herein or are known to those of ordinary skill. The instruction information may be in a computer readable media containing machine-readable instructions that, when executed using a computer, cause the display of a real or virtual procedure of purifying extracellular vesicles from a sample and isolating genomic DNA therefrom, expressing a recombinant protein therein, or identifying a cancer cell-derived marker thereon.

The present disclosure is further illustrated, without being limited to, the examples below.

### EXAMPLES

### A. Materials and Methods

### A.1. Patients and animals

Human samples were obtained from patients with histologically confirmed sarcoma upon validation by board-certified pathologists. Samples were included in our clinical biobank after the written agreement of patients and approval by the local Ethics Committee and the national agency for drug safety (Agence Nationale de Sécurité du Médicament). For mouse experiments, the size of the experimental groups was calculated on the basis of previous experiments to reach a statistical test power of 0.80 and a significance of 0.05. Groups of mice were randomized by the size of the tumor at the beginning of the experiment. Experiments were performed in accordance with protocols approved by the French Council of Animal Care guidelines and national ethical guidelines of the Institut National de la Santé et de la Recherche Médicale (INSERM) Animal Care Committee.

### A.2. Cell lines

Cell lines were purchased from ATCC and cultured following recommendations. Cell culture reagents were purchased from Gibco (Invitrogen). Briefly, cells were kept at 37 °C in a humidified 5% CO2 incubator and maintained in medium supplemented with 10% FBS (Hyclone, Thermo Scientific) and 1% Penicilline/Streptavidin (Invitrogen).

### A.3. Xenografting mice

Mice liposarcoma PDX models were established in collaboration with the surgical and pathology departments of the "Institut du Cancer de Montpellier" (ICM) by inserting a human tumor fragment of approximately 40 mm3 subcutaneously on 8-week-old nude mice (Charles River). Volumetric measurements of xenografted tumors were performed every 3 days by the same person using a manual caliper (volume = length × width). All animals were euthanized when the first animal reached the ethical endpoint (volume = 1000 cm3 or ulceration).

### A.4. Preparation of platelets

Platelets were prepared from patient samples using RegenKit^{®} A-PRP (RegenLab) according to manufacturer recommendations.

### A.5. Preparation of extracellular vesicles

Extracellular vesicles were prepared from cell culture supernatants or patient sera by sequential centrifugation. For cell culture supernatants specifically, a first centrifugation (300g for 5 min at 4°C) served to remove dead cell and another one (3000g for 30 min at 4°C) to remove apoptotic bodies. Then, for all samples, a first ultracentrifugation (10000g for 30 min at 4°C) was processed to remove macrovesicles. A final ultracentrifugation (100000g for 180 min at 4°C) allowed to purification of extracellular vesicles which were resuspended in endotoxin-free PBS and stored at -80°C in low binding microtubes.

### A.6. Sizing the extracellular vesicles

Extracellular vesicles were analyzed using a NanoAnalyzer (ZetaView, Particle Metrix). Parameters such as size distribution, concentration and zeta potential were obtained following manufacturer recommendations.

### A.7. Quantification of MDM2-encoding DNA

Total DNAs from platelets or extracellular vesicles were prepared using TriReagent (Invitrogen) or Quick-DNA HMW MagBead Kit (Zymo). Real-time quantitative PCRs were performed on a LightCycler 480 apparatus (Roche) using the Platinum Taq DNA polymerase (Invitrogen) and a SYBR Green mix containing 3 mM MgCl2 and 30 µM of each dNTP using the following amplification procedure: 45 cycles of 95°C for 10 s, 60°C for 10 s, and 72°C for 20 s. Primers specific of MDM2 intron 11 were used (cf table of sequences). The relative DNA copy number was calculated using Ct value.

### A.8. Quantification of IL-6 protein

IL-6 concentration was measured either from total platelets or from supernatants of activated platelets by an immunoassay (Human IL-6 Standard TMB ELISA Development Kit Catalog Number: 900-T50) following the manufacturer's instructions. Platelets were activated by adding calcium chloride for 30 min. After centrifugation, platelet supernatants were subjected to IL-6 measurement.

### Example 1 : Presence of MDM2-encoding DNA in chromatin-bound MDM2 cancers

Quantification of human MDM2 DNA was performed in mouse organs originating (i) from mice xenografted with a human liposarcoma tumor having chromatin-bound MDM2 and (ii) from mice xenografted with a human ovarian cancer.

The results are depicted in **Figure 1****.**

The results show that MDM2-encoding DNA is found in both types of cancers. Thus, quantifying MDM2-encoding DNA in tumor cells does allow discriminating between (i) chromatin-bound MDM2 cancers.

Further, the results of **Figure 1** also show that a detectable but low amount of MDM2-encoding DNA is found in the muscles but not in the other organs (spleen, stomach, liver, brain, ling, pancreas, intestine and kidney).

Further, the results of **Figure 2** show that in mice that have been xenografted with two different liposarcoma cell lines (IB111 and IB115, respectively) MDM2-encoding human DNA was found mainly in the tumor cells, with a low amount of MDM2-encoding DNA also found in muscles (gastroleus, tibialis, soleus and quadriceps), whereas no MDM2-encoding DNA was found in the other organs (spleen, stomach, liver, brain, ling, pancreas, intestine and kidney).

The content in MDM2-encoding DNA was also assessed in extracellular vesicles derived (i) from human liposarcoma cells having chromatin-bound MDM2 (IB115 cell line) and (ii) from human ductal carcinoma cells having a MDM2 amplification but devoid of chromatin-bound MDM2 (ZR75.1 cell line).

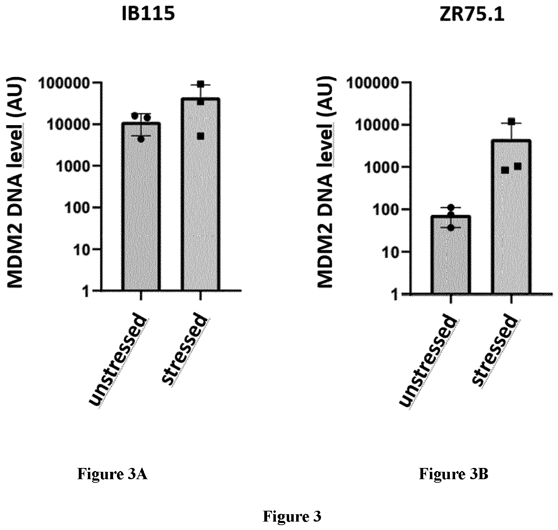

The results are depicted in **Figure 3****.**

Both cancer cell lines have been treated so as to cause a stress inducing localization of MDM2 to the chromatin. As shown in **Figure 3****,** significant MDM2-encoding DNA is found to be inducible by stress in the extracellular vesicles for both cancer cell lines, the liposarcoma line IB115 (cMDM2) as well as the ductal carcinoma ZR75.1 (non cMDM2) (right bar of each of figures 2A and 2B). This results means that a non cMDM2 cancer can be induced to the cMDM2 status following a stress like chemotherapy, radiotherapy and thus can be diagnosed and treated as cMDM2 after such a stress.

Both cancer cell lines have been treated so as to cause a stress inducing localization of MDM2 to the chromatin. As shown in **Figure 3****,** significant MDM2-encoding DNA is found in the extracellular vesicles for both cancer cell lines (right bar of each of figures 3A and 3B).

These results show that discrimination between cancers having MDM2 localized at the chromatin and cancers devoid of chromatin-bound MDM2 can be made by quantifying MDM2-encoding DNA in extracellular vesicles thereof.

### Example 2 : human MDM2-encoding DNA and human IL-6 in extracellular vesicles derived from human cancer cells of cancers having chromatin-bound MDM2

Human MDM2-encoding DNA has been sought in extracellular vesicles obtained from platelets present the tumor interstitial fluids or from the blood platelets of human subjects with cancer cells having chromatin-bound MDM2, *i.e.* human liposarcoma cells.

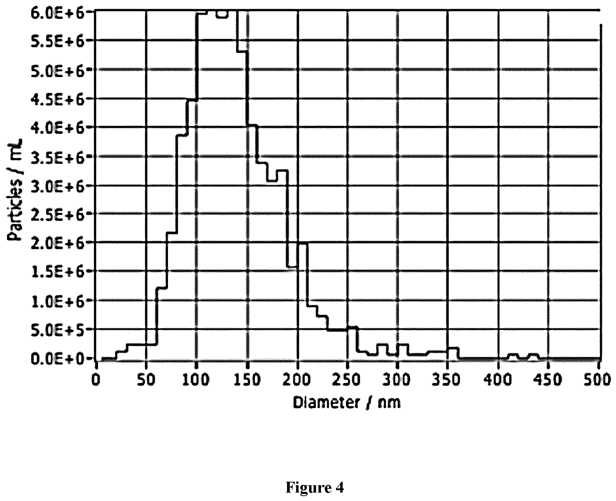

As shown in **Figure 4****,** these extracellular vesicles have a diameter size range between about 50 nm to about 250 nm.

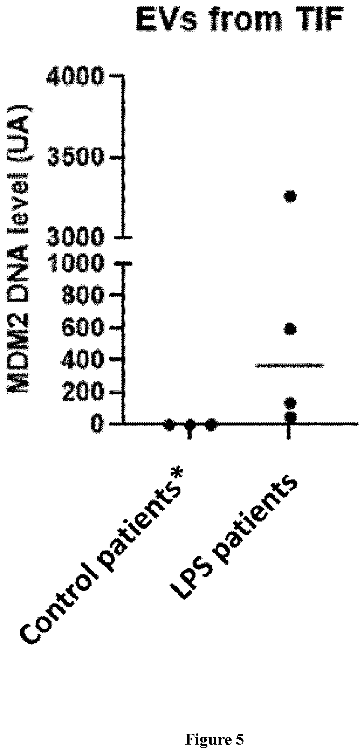

As shown in **Figure 5****,** MDM2-encoding DNA was found only in the extracellular vesicles derived from the tumor interstitial fluids from subjects affected with a cancer having chromatin-bound MDM2. No MDM2-encoding DNA was found in the tumor interstitial fluids collected from patients having a cancer devoid of chromatin-bound MDM2/

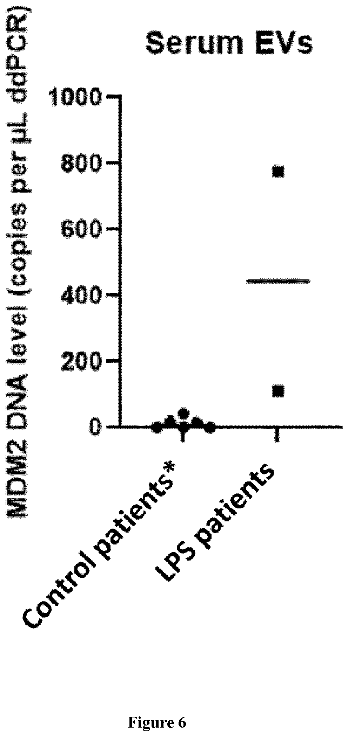

As shown in **Figure 6****,** MDM2-encoding DNA was found only in the extracellular vesicles derived from the blood from subjects affected with a cancer having chromatin-bound MDM2. No MDM2-encoding DNA was found in the blood collected from patients having a cancer devoid of chromatin-bound MDM2.

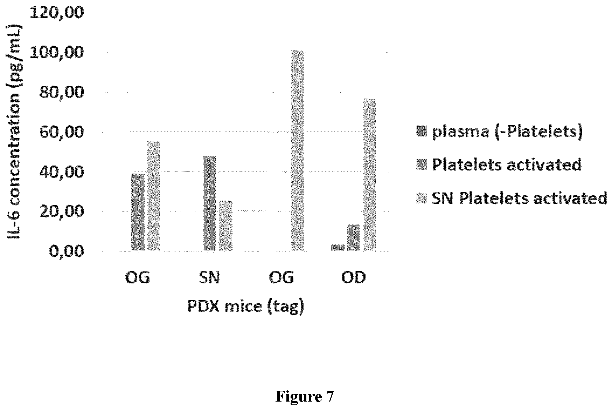

Further, it is shown in **Figure 7** that supernatants of activated platelets derived from the blood of mice xenografted with a chromatin-bound MDM2 cancer, *i.e.* a human liposarcoma, beyond MDM2-encoding DNA, also contain IL-6 protein.

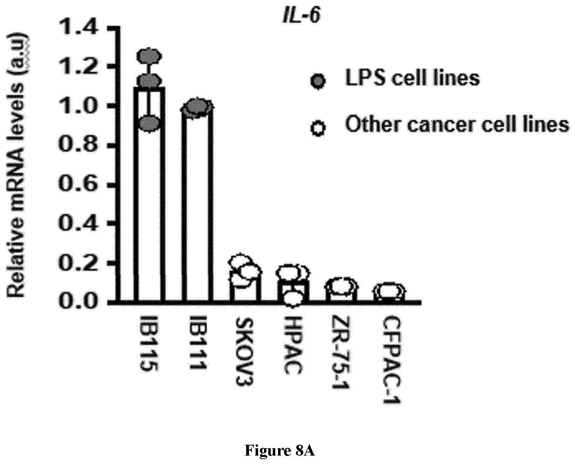

Still further, as shown in **Figure 8A****,** cancer cell lines having chromatin-bound MDM2 *(i.e.* IB115 and IB111 liposarcoma) contain interleukin 6 (IL-6)-encoding mRNA, whereas cancer cell lines devoid of chromatin-bound MDM2 (*i.e.* (i) SKOV3 ovarian adenocarcinoma cell line, (ii) HPAC pancreatic ductal adenocarcinoma cell line, (iii) ZR-75-1 ductal breast cell line and (iv) CFPAC-1 ductal pancreatic adenocarcinoma cell line) do not contain interleukin 6 (IL-6)-encoding mRNA.

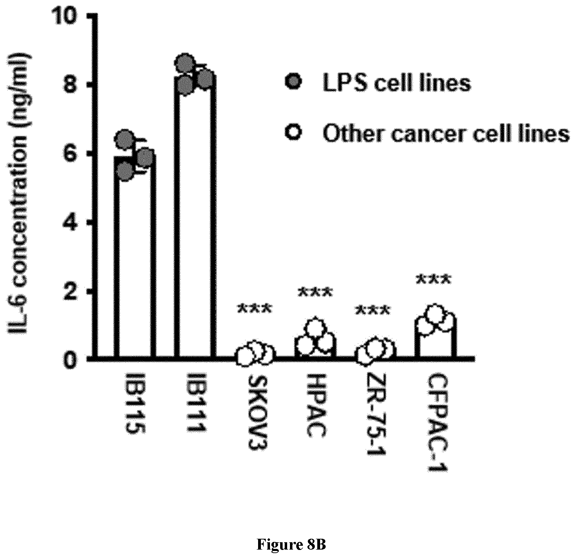

Still further, as shown in **Figure 8B****,** supernatants from cancer cell lines having chromatin-bound MDM2 (*i.e.* IB115 and IB111 liposarcoma) contain interleukin 6 (IL-6) protein, whereas supernatants from cancer cell lines devoid of chromatin-bound MDM2 (*i.e.* (i) SKOV3 ovarian adenocarcinoma cell line, (ii) HPAC pancreatic ductal adenocarcinoma cell line, (iii) ZR-75-1 ductal breast cell line and (iv) CFPAC-1 ductal pancreatic adenocarcinoma cell line) do not contain interleukin 6 (IL-6) protein.

Finally, as it shown in **Figure 9** MDM2-encoding DNA is found in the platelets from blood samples from human subjects affected with a cancer having MDM2 localized at the chromatin (*i.e.* chromatin-bound MDM2 liposarcoma), when compared to platelets from blood samples from healthy human subjects.

### Table of sequences

| **SEQ ID NO.** | **Sequence** | **Description** |
|---|---|---|
| **1** | AATTGAGATAGGGTCCTGCC | human MDM2 forward primer |
| **2** | TGGCATGCACCTGTTATTCC | human MDM2 reverse primer |
| **3** | TTCCAGGCTGGAGTGCAGTGATG | human MDM2 amplicon probe |

## Claims

1. An *in vitro* use of the combined (i) measure of MDM2-encoding DNA and (ii) measure of interleukin 6 protein, in platelets, or in platelet-derived extracellular vesicles, of a subject, as a biomarker indicative of the occurrence of a cancer having chromatin-bound MDM2 in the said subject.

2. An *in vitro* diagnostic method for determining the occurrence of a cancer having chromatin-bound MDM2 in a subject, comprising the steps of:
a) measuring (i) MDM2-encoding DNA and (ii) interleukin 6 protein, in platelets, or in platelet-derived extracellular vesicles from a blood sample from the said subject, wherein the combined measures of MDM2-encoding DNA and of interleukin 6 protein consist of a biomarker indicative of the occurrence of a cancer having chromatin-bound MDM2 in the said subject, and
b) concluding on the occurrence of a cancer having chromatin-bound MDM2 in the said subject, based on the values measured at step a).

3. The *in vitro* diagnostic method according to claim 2, wherein the platelet-derived extracellular vesicles are obtained from a platelet-rich plasma sample or from a platelet-rich plasma sample containing activated platelets.

4. The *in vitro* diagnostic method according to claim 2, wherein the platelet-derived extracellular vesicles are obtained according to a method comprising the steps of:
a) providing platelet-rich blood plasma from a subject's whole blood sample,
b) performing a first ultracentrifugation step, so as to provide a sample depleted in macrovesicles,
c) performing, on the sample obtained at step b), a second ultracentrifugation step so as to provide a sample where extracellular vesicles are present in the pellet thereof, and
d) collecting the extracellular vesicles in the pellet obtained at step c).

5. The *in vitro* method according to claim 2, wherein platelet-derived extracellular vesicles are obtained by a method comprising the steps of:
i) obtaining platelet-rich blood plasma sample from a subject's whole blood sample,
ii) activating the platelets contained in the blood plasma sample obtained at step i), so as to cause the release of the extracellular vesicles contained in the activated platelets in the platelet environment medium, and
iii) purifying the extracellular vesicles contained in the platelet environment medium obtained at step ii).

6. The *in vitro* diagnostic method according to any one of claims 2 to 5, wherein MDM2-encoding DNA is measured by performing a DNA amplification reaction.

7. The *in vitro* diagnostic method according to any one of claims 2 to 5, wherein interleukin 6 protein is measured by performing a method selected from an immunological method, a nuclear magnetic resonance (NMR) method or a method using mass spectrometry.

8. The *in vitro* diagnostic method according to any one of claims 2 to 7, wherein the cancer having chromatin-bound MDM2 is selected among bone cancer, brain cancer, ovary cancer, breast cancer, lung cancer, colorectal cancer, osteosarcoma, skin cancer, malignant hemopathies, pancreatic cancer, prostate cancer and liposarcoma.

9. A therapeutic agent aimed at treating a cancer having chromatin-bound MDM2 for use in the treatment of a subject that has been determined as being affected with cancer having chromatin-bound MDM2 by performing the *in vitro* diagnostic method method according to any one of claims 2 to 7.

10. A therapeutic agent for use according to claim 9, wherein the said therapeutic agent is selected among an inhibitor of the MDM2-p53 interaction and an interleukin 6 signaling inhibitor.

11. A therapeutic agent for use according to claim 9, wherein the said interleukin 6 signaling inhibitor is selected among an anti-IL-6 antibody, an anti-sense oligonucleotide directed to IL-6 mRNA, an anti-IL-6 receptor antibody, an anti-sense oligonucleotide directed to IL-6 receptor mRNA, an anti-IL6/IL-6 receptor complex and a gp130 inhibitor.

12. A therapeutic agent for use according to claim 11, wherein the gp130 inhibitor is selected among an anti-gp130 antibody, bazedoxifene and an anti-sense oligonucleotide directed to gp130 mRNA.

13. A therapeutic agent for use according to claim 9, wherein the said therapeutic agent is a MDM2 inhibitor.

14. A kit for determining the occurrence of a cancer having chromatin-bound MDM2 in a subject comprising (i) means for measuring MDM2-encoding DNA and (ii) means for measuring interleukin 6 protein.
